# EUROPEAN PATENT SPECIFICATION

(11) **EP 3 723 629 B1**
(45) Date of publication and mention of the grant of the patent: **20.11.2024**
(21) Application number: 18887907.6
(22) Date of filing: 13.12.2018
(51) Int. Cl.: A61B 17/12, A61B 17/00, A61B 50/30, A61B 17/04, A61B 90/90, A61B 17/34

(54) **MEDICAL PLUGS, DEVICES FOR DELIVERING THE PLUGS TO VOIDS, AND RELATED KITS**
MEDIZINISCHE STOPFEN, VORRICHTUNGEN ZUR FREISETZUNG DER STOPFEN IN HOHLRÄUMEN UND ENTSPRECHENDE KITS
BOUCHONS MÉDICAUX, DISPOSITIFS DE POSE DES BOUCHONS DANS DES VIDES, ET KITS

(30) Priority: 14.12.2017 US 201762598869 P; 09.08.2018 US 201862716499 P
(43) Date of publication of application: 21.10.2020
(73) Proprietor: MERIT MEDICAL SYSTEMS, INC., South Jordan, UT 84095 (US)
(72) Inventor: SYKES, Kenneth, Bluffdale, Utah 84065 (US); MARION, Pierre, 93360 Neuilly Plaisance (FR); MOTTOLA, Jim, West Jordan, Utah 84088 (US); JENKINS, Richard P., Bluffdale, Utah 84065 (US)
(74) Representative: Ricker, Mathias
(86) International application number: PCT/US2018/065495
(87) International publication number: WO 2019/118747

(56) References cited:
- WO-A1-2017/173378
- WO-A2-2006/119256
- WO-A2-2011/057282
- US-A1- 2001 031 948
- US-A1- 2005 124 999
- US-A1- 2006 099 238
- US-A1- 2016 262 737
- US-A1- 2017 281 151
- US-B2- 8 915 941

## Description

### RELATED APPLICATIONS

This application claims priority to United States Provisional Application No. 62/598,869 filed December 14, 2017, and titled "Medical Plugs and Devices for Delivering the Plugs to Voids," and U.S. Provisional Application No. 62/716,499 filed August 9, 2018, and titled "Medical Plugs, Devices for Delivering the Plugs to Voids, and Related Kits and Methods".

### TECHNICAL FIELD

The present disclosure relates generally to the field of medical devices. More particularly, some embodiments relate to a medical plug, such as a pledget, and medical devices for delivering the medical plug to at least partially fill a void in a patient, and kits for packaging such medical devices. Related components are also disclosed. The document WO 2011/057282 A2 describes a medical deployment instrument forming part of a vessel closure system. The document US 2001/031948 A1 describes a system for creating a mechanical barrier to seal a vascular puncture site, the components of the system prepackaged in a site access kit and a barrier component kit. US 2017/0281151 A1 describes medical devices for delivering a plug to a void within a patient.

### SUMMARY OF THE INVENTION

The invention is defined in independent claim 1. Certain optional features of the invention are defined in the dependent claims. The methods described herein do not form part of the invention.

### BRIEF DESCRIPTION OF THE DRAWINGS

The written disclosure herein describes illustrative embodiments that are nonlimiting and non-exhaustive. Reference is made to certain of such illustrative embodiments that are depicted in the figures, in which:
FIG. 1 illustrates a perspective view of a medical device, according to an embodiment.
FIG. 2 illustrates a front view of the plug of the medical device of FIG. 1.
FIG. 3 illustrates a side view of the plug of FIG. 2.
FIG. 4 illustrates a front view of a plug according to an embodiment.
FIG. 5 illustrates a front view of a plug according to an embodiment.
FIG. 6 illustrates a front view of a plug according to an embodiment.
FIG. 7 illustrates a side view of the plug holder of FIG. 1, according to an embodiment.
FIG. 8 illustrates a front view of the plug holder of FIG. 7, according to an embodiment.
FIG. 9 illustrates a cross-sectional view of the plug holder along cross-section line of 9-9 of FIG. 8, according to an embodiment.
FIG. 10A illustrates a cross-sectional view of the plug holder along cross-section line 10-10 of FIG. 8, according to an embodiment.
FIG. 10B illustrates a cross-sectional view of the plug holder along cross-section line 10-10 of FIG. 8, according to another embodiment.
FIG. 10C illustrates a cross-sectional view of the plug holder along cross-section line 10-10 of FIG. 8, according to another embodiment.
FIG. 11 illustrates a perspective view of the plug holder of FIG. 7 and a preliminary stylet that engages the plug within the plug holder, according to an embodiment.
FIG. 12 illustrates a cross-sectional view of the plug holder and the preliminary stylet of FIG. 11 and a coaxial introducer, according to an embodiment.
FIG. 13 illustrates a cross-sectional view of the plug holder of FIG. 11 attached to a coaxial introducer with a stylet about to engage the plug.
FIG. 14 illustrates a cross-sectional view of the plug holder of FIG. 11 attached to the coaxial introducer and the stylet engaging the plug.
FIG. 15 illustrates a side view of a medical device configured to deliver a plug through a catheter by a syringe.
FIG. 16 illustrates a side view of the medical device of FIG. 15 with the plug delivered through the catheter by the syringe.
FIG. 17 illustrates a perspective view of a kit including a flexible package with various components disposed therein.
FIG. 18 illustrates an exploded view of a stylet assembly with a protective cover.

### DETAILED DESCRIPTION

Certain medical procedures include delivery of a plug (such as a pledget) into a void within a patient's body. Plugs may be inserted into a void to, inter alia, partially or completely fill a wound site, to occlude the passage of fluid through a lumen, to induce blood coagulation, to prevent or reduce leakage of biological fluid, and/or to provide a scaffold to promote and/or permit tissue growth.

For instance, during a biopsy procedure, a practitioner may insert a coaxial introducer into a patient by placing a trocar within a coaxial introducer such that a pointed distal end of the trocar protrudes from the distal end of the coaxial introducer. With the pointed end of the trocar protruding from the coaxial introducer, the trocar and the coaxial introducer may together be inserted into the patient. Once the coaxial introducer is positioned within the patient, the trocar may be withdrawn from the coaxial introducer. At this stage of the procedure, the coaxial introducer provides a conduit that allows access to a patient's internal tissue.

A cutting device (e.g., a needle or some other device configured to obtain bodily samples) may then be inserted through the coaxial introducer. Once the cutting device reaches the internal tissue, the cutting device may be used to excise (e.g., cut out) internal tissue from the patient. Such excision may leave behind a void in the space that was occupied by the internal tissue.

In some circumstances, a practitioner may desire to deliver a plug into the void created by excised tissue from the biopsy procedure. For example, in some embodiments, a plug may be inserted into the void to at least partially fill the space created by the void, to promote blood coagulation at the wound site, and/or to provide a scaffold to promote or permit tissue regrowth.

Plugs may be inserted into a void in other medical procedures as well. For example, a plug may be delivered to block fluid flow through a lumen. In other words, a plug may be delivered as an embolic agent to prevent the flow of fluid to a particular location. Plugs may be delivered to various other locations in a patient's body, or may be delivered under alternative circumstances or for different purposes. This disclosure relates broadly to the delivery of plugs for various purposes, and is not limited to the specific contexts or examples discussed herein.

Medical devices and related components, as described in greater detail below, may be configured to facilitate delivery of a plug into a void. In some circumstances, the medical devices are designed to facilitate wetting (e.g., hydration) of a plug and subsequent delivery of the plug through a lumen to a void within a patient.

Medical devices may be packaged to protect against environmental conditions associated with transportation and handling from the time of manufacture until use by a medical practitioner. Such protections may be configured to facilitate proper function of the device when it is eventually used. For example, a package or enclosure may be designed to shield the medical device from environmental conditions that otherwise might damage the medical device or otherwise render it unusable. In some instances, packaging materials may be used to maintain the sterility of a sterile medical device by creating a sterile barrier such as a sealed flexible pouch. Additionally, mechanical shock and vibration associated with handling and transportation of a medical device may also damage the medical device. Secondary packaging such as foam or other shock absorbing materials may be used in connection with a primary package (such as a flexible pouch) to prevent such damage. As described below, in some embodiments a primary package may be configured to both seal and provide shock absorption to enclosed medical devices.

The components of the embodiments as generally described and illustrated in the figures herein can be arranged and designed in a wide variety of different configurations. Thus, the following more detailed description of various embodiments, as represented in the figures, is not intended to limit the scope of the present disclosure, but is merely representative of various embodiments. While various aspects of the embodiments are presented in drawings, the drawings are not necessarily drawn to scale unless specifically indicated.

The phrase "coupled to" is broad enough to refer to any suitable coupling or other form of interaction between two or more entities. Two components may be coupled to each other even though they are not in direct contact with each other. For example, two components may be coupled to each other through an intermediate component. The phrase "fluid communication" is used in its ordinary sense, and is broad enough to refer to arrangements in which a fluid (e.g., a gas or a liquid) can flow from one element to another element when the elements are in fluid communication with each other.

The terms "proximal" and "distal" are opposite directional terms. For example, the distal end of a device or component is the end of the component that is furthest from the practitioner during ordinary use. The proximal end refers to the opposite end or the end nearest the practitioner during ordinary use. The term "void" relates to a region or an opening within a patient's body to which a plug may be delivered.

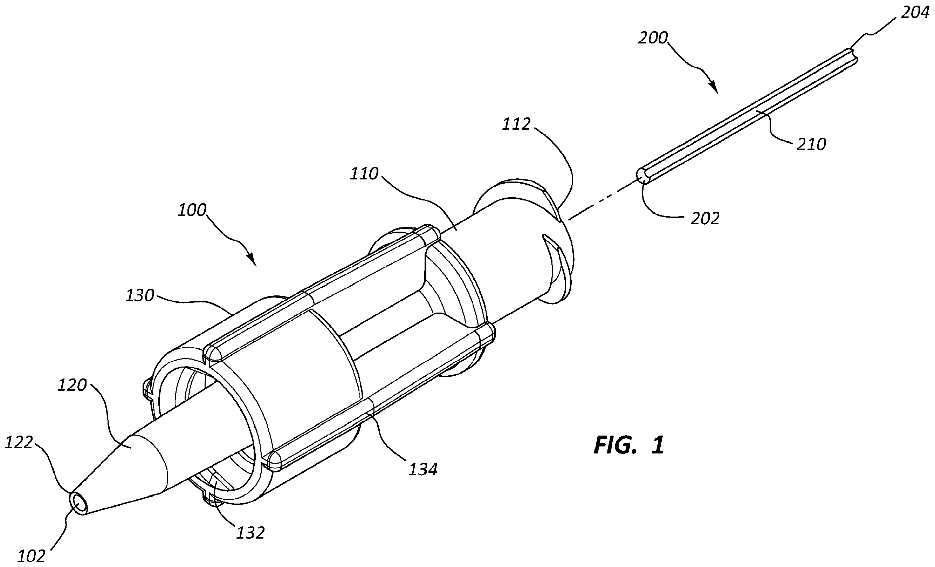

FIG. 1 illustrates a perspective view of a medical device that may include a plug holder 100 (which may also be referred to as a medical plug delivery device or an inline coupler) and a medical plug or pledget 200.

Plug 200 may be generally elongate in shape and have a variety of different cross-sections, such as circular, square, triangular, etc. Plug 200 illustrated in FIG. 1 has a substantially circular cross-section. In some embodiments, plug 200 may have a first cross-section, such as a square, and then be crimped to a second cross-section, such as a circle. In some embodiments, plug 200 is an elongate piece of material that has been rolled into a substantially cylindrical shape between 1 mm and 5 mm (e.g., approximately 2 mm) in diameter. Plug 200 may have a length that is at least 2-fold, at least 5-fold, and/or at least 10-fold longer than the diameter of plug 200. In some embodiments, plug 200 is between 10 mm and 30 mm (e.g., approximately 20 mm) in length.

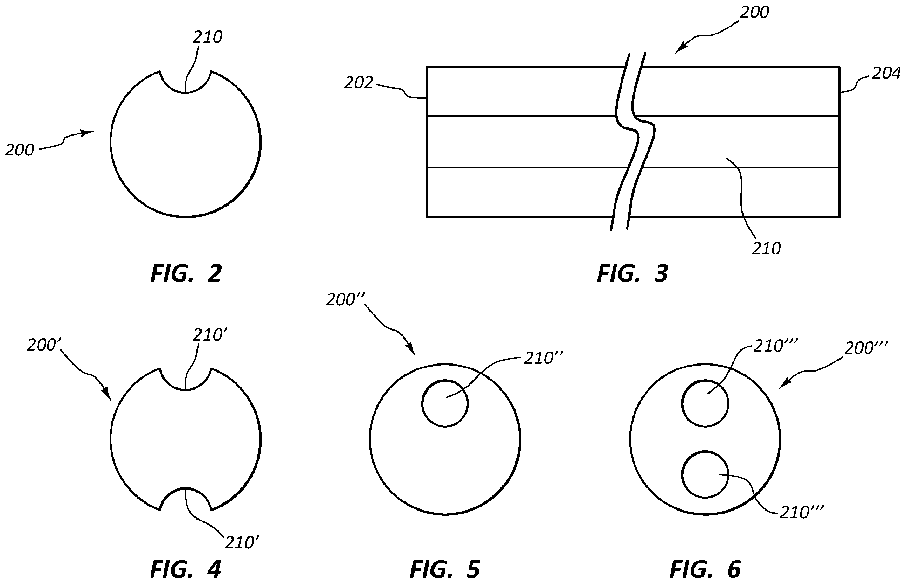

Plug 200 may further include a channel 210 (which may also be referred to as a fluid bypass pathway) along the outer surface of plug 200. Channel 210 may extend from a first end 202 to a second end 204 of plug 200. FIG. 2 illustrates a front view of plug 200 with channel 210. Channel 210 may have an arc shape or a semi-circle cross-section. However, various other shapes may be used. FIG. 3 illustrates a top view of plug 200 and channel 210. As illustrated in FIG. 3, channel 210 may extend the entire length of plug 200, from first end 202 to second end 204. As described in further detail below, channel 210 provides a fluid bypass pathway to enable air and fluid to bypass plug 200 when plug 200 is wetted. The movement of the fluid through channel 210 forces any air within a lumen 102 of plug holder 100 out of plug holder 100. In addition, when plug 200 is wetted, plug 200 may swell thereby closing channel 210.

Channel 210 may be formed by placing a mandrel in plug 200 while plug 200 is formed. Thus, the shape of channel 210 is similar to the shape of the mandrel when the mandrel is removed from plug 200.

FIGS. 4-6 illustrate front views of various other potential embodiments of plugs. The plugs of FIGS. 4-6 may be used in connection with devices or systems described in connection with plug 200 of FIGS. 1-3. Disclosure given in connection with plug 200 analogously may be applied to the plugs of FIGS. 4-6 or vice versa. FIG. 4 illustrates a plug 200' with two channels 210' along the outer surface of plug 200'. Channels 210' may extend from a first end to a second end of plug 200'. Alternatively, plug 200' may have more than two channels, such as three, four, or more. Channels 210' may be equally spaced from each other, or channels 210' may be unequally spaced around the outer surface of plug 200'. Each channel 210' may be an arc shape or a semi-circle cross-section. Various other shapes for channel 210' may be used.

FIG. 5 illustrates a plug 200" with channel 210" wholly disposed within plug 200", giving plug 200" a donut shape. Channel 210" may be centered in plug 200", or channel 210" may be off-center of plug 200". Channel 210" may have a circular cross-section and channel 210" may extend the entire length of plug 200", from a first end to a second end. However, channel 210" may have various other cross-sectional shapes. Plug 200" may also include more than one channel 210" disposed within plug 200". For example, FIG. 6 illustrates a plug 200‴ with two channels 210‴ disposed within plug 200'''. In other embodiments, plug 200‴ may include a channel 210‴ along the outer surface of plug 200‴ (similar to channel 210 of FIG. 2) and a channel 210‴ wholly disposed within plug 200‴ (similar to channel 210' of FIG. 4).

With reference to FIGS. 1-3, plug 200 may be composed of any suitable composition or material. For example, in some embodiments, plug 200 may include, comprise, or consist of a bioabsorbable material. In some embodiments, the bioabsorbable material (or a portion thereof) is derived from animal tissue, such as pig skin or cow skin. In some embodiments, the bioabsorbable material is a collagen-containing material, such as a gelatin foam from an animal source. In other or further embodiments, the bioabsorbable material (or a portion thereof) is a synthetic polymer, such as polylactic acid, polyglycolide, or poly(lactic-co-glycolic acid). In some embodiments, plug 200 includes or consists of a non-bioabsorbable material, such as polyvinyl alcohol or polyvinyl acetate. In some embodiments, plug 200 includes a dye. The dye may facilitate visualization of plug 200 when plug 200 is disposed within plug holder 100. In some embodiments, plug 200 may change colors when contacted with fluid (e.g., water or saline), thereby allowing a practitioner to visually determine when plug 200 has been wetted.

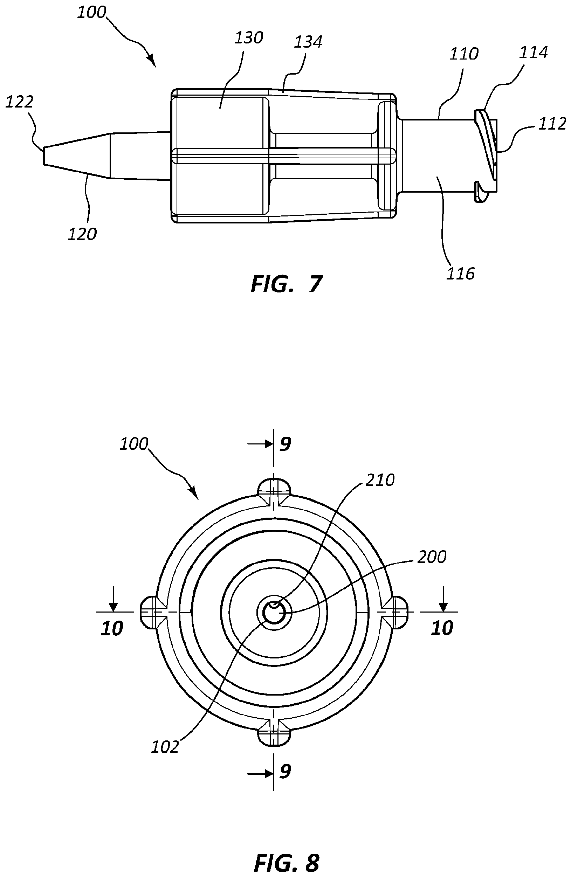

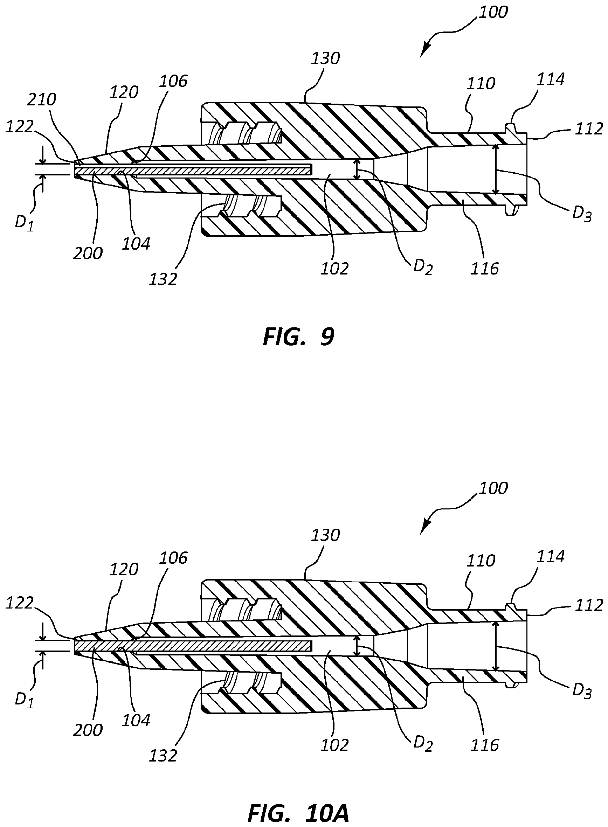

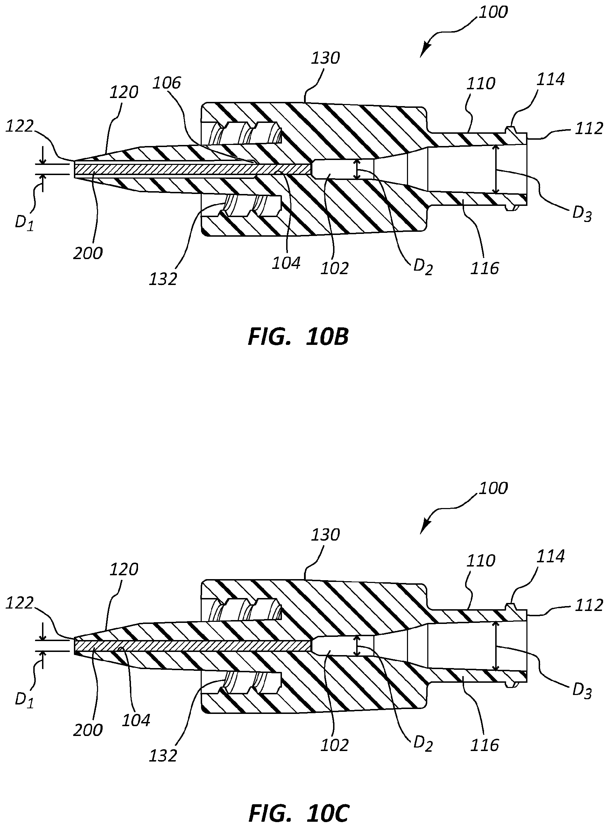

FIGS. 7-10C illustrate various views of plug holder 100 of FIG. 1. FIG. 7 illustrates a side view of plug holder 100. FIG. 8 illustrates a front view of plug holder 100. FIG. 9 illustrates a cross-sectional view of plug holder 100 along cross-section line 9-9. FIG. 10A illustrates a cross-sectional view of plug holder 100 along cross-section line 10-10 according to one embodiment. FIG. 10B illustrates a cross-sectional view of plug holder 100 along cross-section line 10-10 according to another embodiment. FIG. 10C illustrates a cross-sectional view of plug holder 100 along cross-section line 10-10 according to another embodiment.

With reference to FIGS. 1 and 7-10C, plug holder 100 may include a proximal portion 110, a distal portion 120, and a central portion 130. Plug holder 100 may include a lumen 102 configured to pass from a proximal end 112 to a distal end 122. Lumen 102 may be configured to accommodate plug 200. Thus, lumen 102 may define a cavity configured to retain plug 200. Lumen 102 may be centrally disposed within plug holder 100 and may be configured to orient plug 200 within plug holder 100 such that the longitudinal axis of plug 200 is aligned with the longitudinal axis of plug holder 100 or lumen 102. Lumen 102 may be generally tapered from proximal end 112 to distal end 122 with the largest diameter located at proximal end 112 and the smaller diameter located at distal end 122. For example, FIGS. 9 and 10 depict three diameters, D1, D2, and D3. D1 is the smallest and located at distal end 122 and D3 is the largest and located at proximal end 112.

As illustrated in FIG. 10A, lumen 102 may include a plug retention section 104 positioned near the distal end 122. Plug retention section 104 may be interference fit. Plug retention section 104 may be configured to retain plug 200 within lumen 102 until displacement of plug 200 is desired and to permit removal of air from lumen 102. Plug retention section 104 may be a portion of lumen 102 with an inner diameter, e.g., D1, less than the outer diameter of plug 200. For example, the outer diameter of plug 200 may be one gauge size different from plug retention section 104 (such as plug 200 being 20 gauge and the inner diameter of plug retention section 104 being 21 gauge). Plug retention section 104 may be 3 mm to 8 mm in length (for example, approximately 5 mm in length) and be configured to frictionally engage a portion of plug 200. A proximal end of plug retention section 104 may include a chamfer 106 to facilitate passage of a stylet that may engage plug 200.

In the embodiment illustrated in FIG. 10B, the plug retention 104 may be positioned near the proximal end of the plug 200 instead of the distal end of the plug 200. In the embodiment illustrated in FIG. 10C, the plug retention section 104 may extend from the distal end of the plug 200 to the proximal end of the plug 200.

FIG. 8 illustrates a front view of plug holder 100 and plug 200. This view clearly illustrates channel 210 or the fluid bypass pathway. Channel 210 creates a pathway for air disposed inside lumen 102 to escape when fluid is applied to proximal end 112 of lumen 102.

FIGS. 9 and 10A-10C illustrate a cross-sectional view of plug holder 100 based on cross-section line 9-9 and 10-10 provided in FIG. 8. FIG. 9 illustrates channel 210 that provides air a pathway to escape, in accordance with the arrow, when fluid is applied to proximal end 112 of lumen 102. In certain configurations, plug 200 is in frictional contact with lumen 102 at the plug retention section 104, as illustrated in FIGS. 10A-10C. Accordingly, during use, any of the air within the system may be forced out via channel 210 such that fluid exits channel 210. This is indicative the air has been purged from the system.

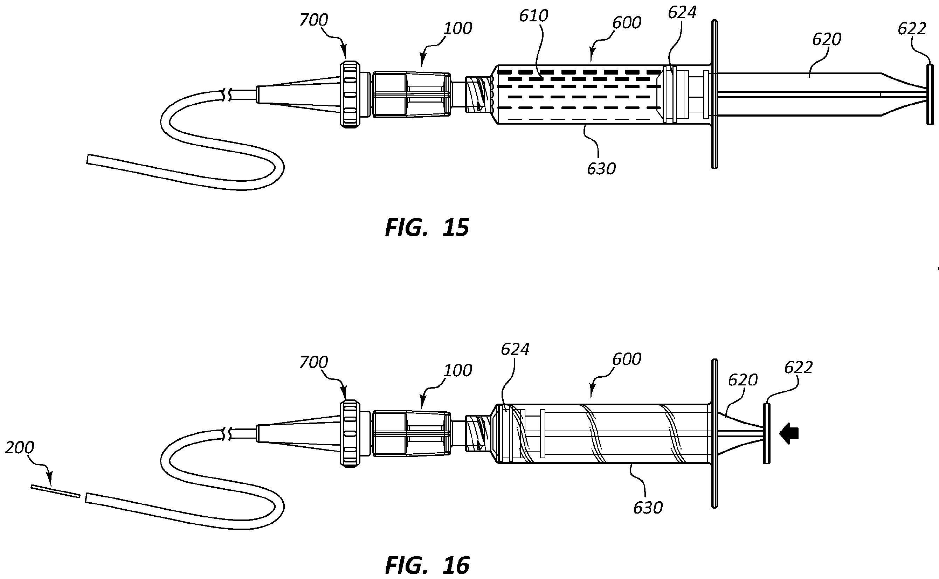

Proximal portion 110 may include a proximal adaptor 114 that is configured to couple to a medical fluid delivery device 600, such as a syringe (see FIGS. 15 and 16). For example, proximal portion 110 may include a female Luer connection that mates with a male Luer connection of the syringe body to form a fluid tight connection (see FIGS. 15 and 16). The female Luer connection may include a female Luer lock fitting 116. Female Luer lock fitting 116 may be configured to sealingly mate with a male Luer lock fitting of a fluid delivery device or other medical device. The outlet or orifice at the distal end of the syringe body may thus be in fluid communication with proximal end 112 of lumen 102.

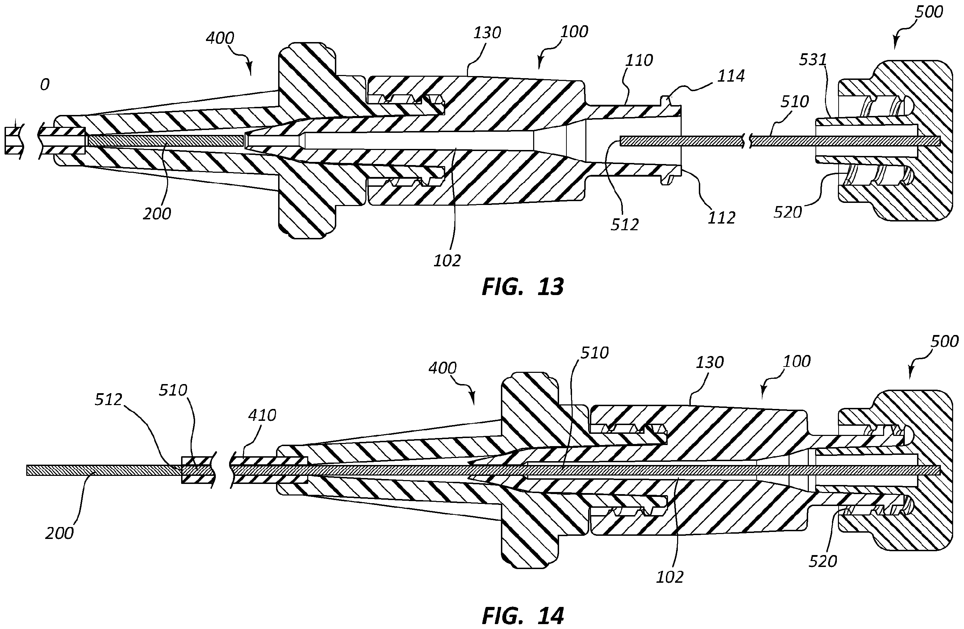

A distal portion of central portion 130 may include a male Luer lock fitting 132. Male Luer lock fitting 132 may be configured to sealingly mate with a female Luer lock fitting of a coaxial introducer 400 which is disposed within a patient (as illustrated in FIGS. 13 and 14) or with a catheter 700 (as illustrated in FIGS. 15 and 16).

Distal portion 120 of plug holder 100 may have a frustoconical-shaped extension within lumen 102 extending through the extension. The extension may extend into coaxial introducer 400 and may or may not create a fluid tight seal with coaxial introducer 400. The extension may be configured to extend lumen distal end 122 to be near and in alignment with the proximal end of the introducer needle allowing for passage of plug 200 from plug holder 100 into coaxial introducer 400 without plug 200 getting caught within coaxial introducer 400 and folding on itself.

Alternative plug holders may be used instead of plug holder 100. For example, plug holders capable of delivering multiple plugs as described in U.S. Patent No. 15/479,149, filed April 4, 2016.

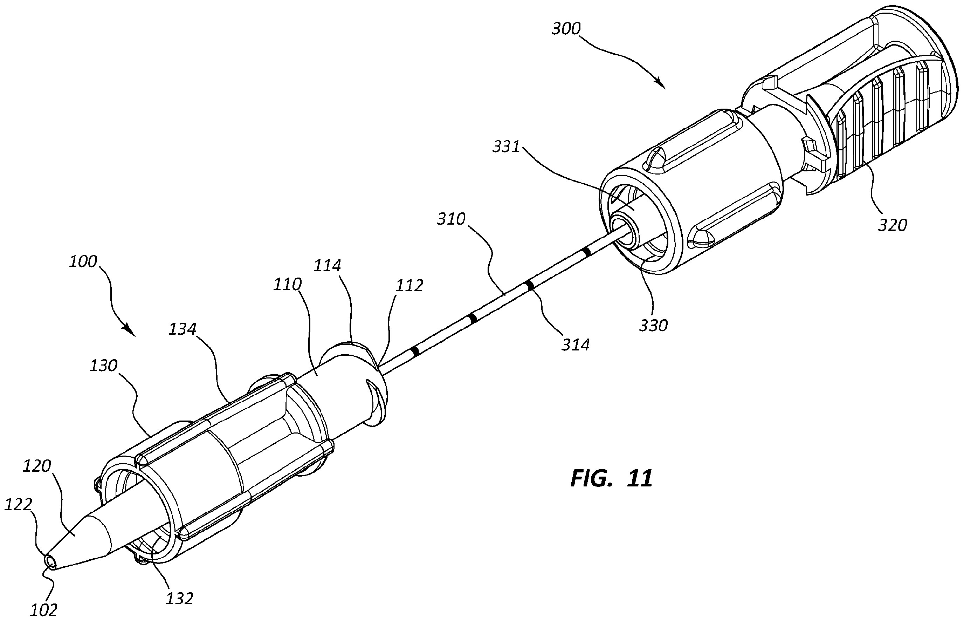

FIGS. 11-16 describe various methods of delivering plug 200 to the patient. The practitioner may select a dry technique or a wet technique for delivering plug 200 into a void of a patient. Plug holder 100 and plug 200 may be interchangeable between dry and wet techniques, enabling the practitioner to select the desired technique when performing the procedure.

In some embodiments, a stylet may be used to introduce a dry plug 200 into the patient, as illustrated in FIGS. 11-14. A practitioner may insert a coaxial introducer 400 into a patient by placing a trocar within coaxial introducer 400 such that a pointed distal end of the trocar protrudes from a distal end of coaxial introducer 400. With the pointed end of the trocar protruding from coaxial introducer 400, the trocar and coaxial introducer 400 may together be inserted into the patient. Once coaxial introducer 400 is positioned within the patient, the trocar may be withdrawn from coaxial introducer 400. At this stage of the procedure, coaxial introducer 400 provides a conduit in an introducer cannula that allows access to a patient's internal tissue. A cutting stylet 500 may be introduced into the conduit of coaxial introducer 400 and a sample of tissue may be excised from the patient, creating a void. The practitioner may then remove cutting stylet 500 and the tissue sample from coaxial introducer 400.

After cutting stylet 500 is removed from coaxial introducer 400, the practitioner may introduce plug 200 into the void where the biopsy was excised. Distal portion 120 of plug holder 100 which houses plug 200 may be connected or attached to the proximal end of coaxial introducer 400. A preliminary stylet 300 may be configured to push or displace plug 200 from plug holder 100 into coaxial introducer 400 or alternatively, directly into the patient.

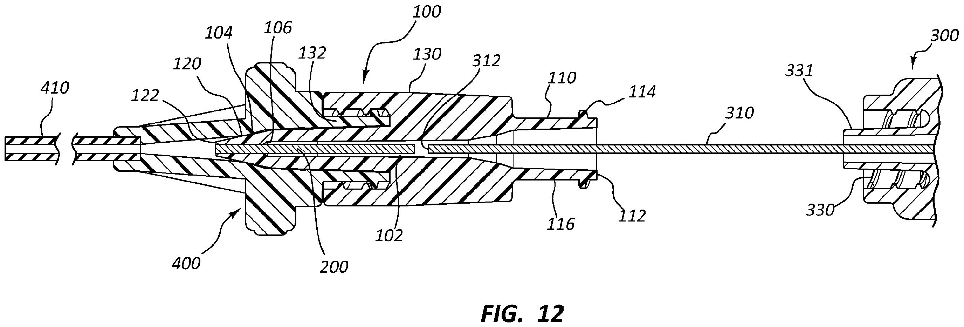

FIG. 11 illustrates a perspective view of plug holder 100, a preliminary stylet 300, and coaxial introducer 400. Preliminary stylet 300 may include a stylet rod 310 and a handle 320 or finger grip. Stylet rod 310 may be metal or rigid plastic, such as polycarbonate. Stylet rod 310 may be configured to pass through lumen 102 from proximal end 112 to distal end 122 and into coaxial introducer 400. A distal end 312 of stylet rod 310 may be squared-off to provide a flat surface to push against plug 200, as illustrated in FIG. 12. As stylet rod 310 is advanced through lumen 102 of plug holder 100, distal end 312 of stylet rod 310 may make contact with the proximal end of plug 200. As a distally directed force is applied to plug 200 by stylet rod 310, the force may exceed the frictional engagement force between plug 200 and lumen retention section 104 of plug holder 100. As the frictional engagement force is surpassed, plug 200 will be displaced from lumen 102 into coaxial introducer 400.

The diameter of stylet rod 310 may be sized to pass through plug retention section 104 of lumen 102 as well as into coaxial introducer 400. The length of stylet rod 310 may be sized to extend through plug holder 100 and partially into the conduit of coaxial introducer 400 for displacement of plug 200 into the conduit of coaxial introducer 400. Alternatively, stylet rod 310 may have a length that enables stylet rod 310 to push plug 200 through conduit 410 of coaxial introducer 400 and into the void of the patient. Stylet rod 310 may include insertion depth markings 314. Markings 314 may be single or multiple bands spaced approximately 1 cm apart. The practitioner may determine the length of stylet rod 310 needed to displace plug 200 into the desired location within the void of the patient or within coaxial introducer 400.

Preliminary stylet handle 320 may be configured to be held between the thumb and at least one finger of the practitioner. Handle 320 may comprise features to enhance gripability, such as roughed surface, ribs, detents, and other features known in the art. Handle 320 may be configured to connect to proximal adaptor 114 of plug holder 100. The distal portion of handle 320 may be structured as a male Luer lock or Luer slip adaptor 330 comprising a distal protrusion 331. Distal protrusion 331 of handle 320 may also be used to interface with and couple to a protective cover (not shown) for stylet rod 310.

In some embodiments, once plug 200 is properly introduced in conduit 410 of coaxial introducer 400, the practitioner may use cutting stylet 500 to place plug 200 into the void within the patient. Since cutting stylet 500 excises the tissue sample, cutting stylet 500 is the proper length to introduce plug 200 to the void within the patient. Cutting stylet 500 may comprise a handle 520, a male Luer lock or Luer slip adaptor 530 and a distal protrusion 531.

In some embodiments, the practitioner may select a wet medical plug delivery technique, as illustrated in FIGS. 15 and 16. Proximal adaptor 114 of plug holder 100 may be connected to a fluid delivery device 600, such as a syringe at least partially filled with a fluid 610. Fluid delivery device 600 may deliver fluid 610 into lumen 102 of plug holder 100 such that lumen 102 becomes free of air and plug 200 is wetted. In some embodiments, plug holder 100 is transparent, thereby allowing the practitioner to visualize wetting and ejection of plug 200. Fluid delivery device 600 may deliver a fluid 610 such as water, saline, contrast, or any mixture thereof, or any other fluid. If desired, air may be removed from lumen 102 by orienting plug holder 100 such that distal end 122 of plug holder 100 is pointed upward, tapping plug holder 100, and ejecting air bubbles by delivering fluid 610 through plug holder 100. Channel 210 of plug 200 permits the passage of fluid 610 through plug 200 and plug retention section 104 to allow air to escape. Once plug 200 is wetted, plug 200 may swell, thus closing channel 210.

Male Luer lock fitting 132 may be connected to a female Luer lock connector of catheter 700 that has been inserted into a patient, as illustrated in FIG. 15. The connection may bring lumen distal end 122 close to alignment with the proximal end of catheter 700. The practitioner may then eject fluid 610 from fluid delivery device 600, thereby distally displacing fluid 610 into lumen 102. As fluid 610 is displaced in a distal direction, fluid 610 may exert a distal force on plug 200 disposed within lumen 102. The hydraulic force may surpass the engagement force of plug retention section 104 on a portion of plug 200, thereby causing distal displacement and ejection of plug 200 from lumen 102 of plug holder 100 into catheter 700 that is in fluid communication with a void of the patient. As fluid 610 is advanced, displaced fluid 610 may push plug 200 through catheter 700 into the void of the patient, as illustrated in FIG. 16. Plug 200 may serve any suitable purpose, such as obstructing fluid flow, inducing blood coagulation, and/or providing a scaffold to promote tissue growth.

Fluid delivery device 600 may include a plunger 620 and a syringe body 630. Plunger 620 may include a handle 622 adjacent the proximal end of plunger 620 and a seal 624 adjacent the distal end of plunger 620. Plunger 620 may be configured to be at least partially disposed within syringe body 630 such that advancement and retraction of plunger 620 causes displacement of fluid 610 within a reservoir in syringe body 630.

Accordingly, the practitioner may select which technique to use at the time of the procedure as plug holder 100 and plug 200 are interchangeable between wet and dry techniques.

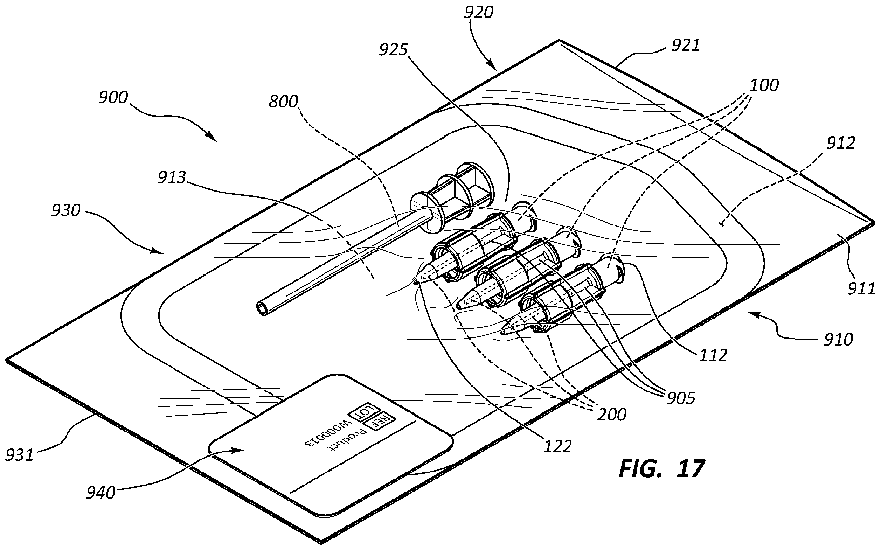

FIG. 17 illustrates a kit 900 for a plurality of medical plug delivery systems 905. Each medical plug delivery system 905 may comprise a medical plug delivery device 100 having an elongate medical plug 200 disposed within the lumen of medical plug delivery device 100 at a distal end thereof. Thus, in the illustrated embodiment, the medical plug delivery system 905 is comprised of one or more medical plug delivery devices 100 as described in connection with certain embodiments above. In other embodiments, the medical plug delivery system 905 may comprise other medical dvices in addition to, or in place of, the medical plug delivery devices 100 described above. Kit 900 may comprise an enclosure, such as sealed enclosure 910 within which one or more medical plug delivery systems 905 may be disposed. Other components may be included in kit 900 and disposed within sealed enclosure 910, such as additional medical plug delivery systems 905, a stylet 800, and a fluid delivery device 600 (not shown). Sealed enclosure 910 may initially be unsealed so as to accommodate disposition of the contents within sealed enclosure 910. Sealed enclosure 910 may be subsequently sealed or otherwise closed to maintain the position of the medical plug delivery devices 100 within the sealed enclosure 910. Embodiments wherein the sealed enclosure 910 is sealed 910 to maintain sterility as well as embodiments wherein the sealed enclosure 910 is configured to simply maintain the grouping of medical plug delivery devices 100 (not necessarily sealed with respect to sterility) are both within the scope of this disclosure.

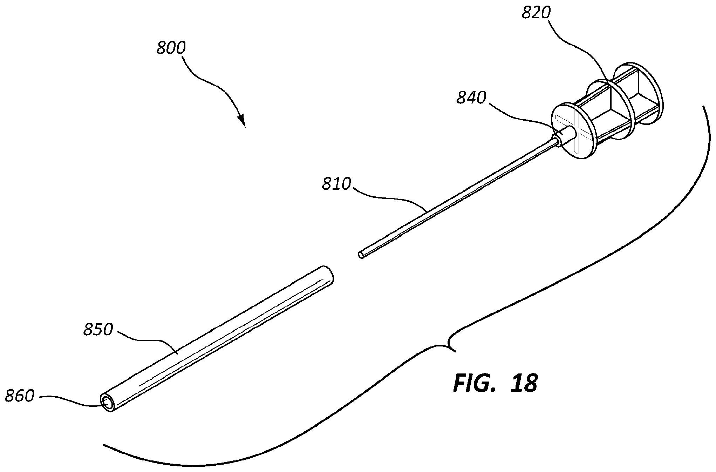

Referring to FIG. 18, stylet 800 may comprise a stylet rod 810 coupled to a stylet handle 820. Stylet handle 820 may comprise a protrusion 840 extending from stylet handle 820 along a portion of stylet rod 810. Stylet 800 may comprise a cover 850 configured to protect stylet rod 810. Cover 850 may be formed in a tubular shape having an internal diameter larger than the outside diameter of stylet rod 810. The outer dimensions of protrusion 840 and the inner diameter of cover 850 may be configured to be releasably coupled together.

Other embodiments of stylets such as preliminary stylet 300 and cutting stylet 500 (such as those described in connection with the embodiments above, such as in FIGS. 11-14) may be included in kit 900 and disposed within sealed enclosure 910. Preliminary stylet 300 and cutting stylet 500 may also comprise tubular protective covers having internal dimensions configured to couple to distal protrusions 331 and 531 respectively.

Kit 900 may comprise human and/or machine-readable indicia 940. Indicia 940 may be printed directly on sealed enclosure 910 or printed on a label adhesively applied to sealed enclosure 910. Indicia 940 may include product identification, manufacturing information (for example date and/or serial number), instructions for use, a shelf life expiration date, etc. Indicia 940 may further include other information as may be contemplated by one of ordinary skill having the benefit of this disclosure.

Sealed enclosure 910 may comprise an envelope or enclosure which may be comprised of flexible walls, such as opposing flexible walls 911. Opposing flexible walls 911 may be sealed or otherwise coupled together along at least a portion of a perimeter. A seal 912 may be disposed inward from the perimeter of opposing flexible walls 911 along at least a portion of the perimeter. As noted above, this seal 912 may comprise a seal configured to maintain sterility, such as a hermetic seal, or may be a coupling region configured to maintain the positions of medical devices within the sealed enclosure, but not necessarily sealed with respect to sterility. Opposing flexible walls 911 may be formed from a single film folded along an edge, a tubular structure or separate films. Opposing flexible walls 911 may have the same or different shapes, thicknesses and materials, and the materials may be compatible with sterilization techniques. One or both opposing flexible walls 911 may be transparent or translucent. Seal 912 may be peelable and the shape of seal 912 may comprise a shape feature to facilitate ease of peeling such as a chevron. Sealed enclosure 910 may be sealed using RF welding, heat welding, or any other suitable method. Sealed enclosure 910 and the contents within may be sterilized using radiation, E-Beam or other methods suitable for sterilizing hermetically sealed enclosures.

As shown, the medical plug delivery system 905 may be disposed at a central region 925 of sealed enclosure 910. The medical plug delivery system 905 may be oriented such that a longitudinal axis of one or more medical plug delivery devices 100, or other elements, are aligned with a longitudinal axis of sealed enclosure 910.

Sealed enclosure 910 may comprise a proximal portion 920 and a distal portion 930. The proximal direction of sealed enclosure 910 may correlate to the proximal direction of medical plug delivery device 100 disposed therein in embodiments wherein the medical plug delivery devices 100 of the medical plug delivery system 905 are aligned and oriented in the same direction as shown in illustrated embodiment. Similarly the distal direction of the sealed enclosure 910 may be defined by the distal direction of medical plug delivery device 100 when arranged as shown in the illustrated embodiment. Proximal portion 920 is defined as the portion of sealed enclosure 910 between proximal end 112 of medical plug delivery device 100 and a proximal end 921 of opposing flexible walls 911. Similarly, distal portion 930 is defined as the portion of sealed enclosure 910 between distal end 122 of medical plug delivery device 100 and a distal end 931 of opposing flexible walls 911.

As described above, elongate medical plug 200 may be frictionally coupled to medical plug delivery device 100 so as to establish an engagement force holding the elongate medical plug 200 within the medical plug delivery device 100. The engagement force may be configured to maintain the position of the elongate medical plug 200 until the elongate medical plug 200 is displaced by a medical practitioner. Thus, the engagement force may be configured to withstand environmental conditions such as vibration and/or shock conditions related to handling and/or transporting of medical plug delivery system 905. In certain instances, however, the vibration and/or shock conditions may produce acceleration levels of the medical plug delivery device 100 sufficient to subject the elongate medical plug 200 to forces great enough to overcome the engagement force, resulting in displacement of elongate medical plug 200 relative to medical plug delivery device 100. In some embodiments, the packaging designs and techniques as described herein may be used to isolate medical devices within the sealed enclosure 910 from vibration or shock, such as by limiting acceleration levels of medical plug delivery device 100 so as to further prevent or limit displacement of elongate medical plug 200 relative to medical plug delivery device 100.

In some instances, the environmental conditions which produce acceleration levels sufficient to displace the elongate medical plugs 200 may occur while a device is disposed within a package. For example, acceleration may occur when a package is dropped on a solid floor or at other times during transportation in a delivery truck. In certain instances, the use of secondary packaging materials may be employed to shield the package contents from such acceleration. The secondary packaging materials establish compliance between the package contents and external contact points of the package. The required level of compliance may be determined and/or verified empirically through shake and drop testing.

As noted herein in certain embodiments, the primary packaging design (such as the sealed enclosure 910) and techniques as described herein may be used to provide compliant package portions to prevent of the packaging contents from being exposed to high acceleration levels without the addition of secondary compliant packaging materials such as foam, bubble wrap, etc.

Sealed enclosure 910 as described herein may be used to create compliance between medical plug delivery system 905 and a contact surface such as a floor, inside wall of a box, etc. The addition of such compliance may prevent vibration, shock, and high acceleration levels from reaching medical plug delivery system 905. In some instances, sealed enclosure 910 may be configured to provide compliance in a specific direction relative to medical plug delivery system 905. As the engagement force between elongate medical plug 200 and medical plug delivery device 100 is in the longitudinal direction, sealed enclosure 910 may be configured to provide additional compliance in the longitudinal direction, for example.

Proximal and distal portions 920, 930 may define compliance, i.e. cushioning, in the respective longitudinal directions of medical plug delivery device 100. Proximal and distal portions 920, 930 may be configured to provide a predetermined compliance level. The level of compliance sufficient for prevention of displacement of elongate medical plug 200 relative to medical plug delivery device 100 may be determined and/or verified empirically through shake and drop testing.

In some instances, the compliance mechanism of proximal and distal portions 920, 930 may be deformation, such as bending and/or buckling, of proximal and distal portions 920, 930. The tendency for longitudinal deformation of proximal and distal portions 920, 930 may be defined by one or several dimensions or shapes of proximal and distal portions 920, 930. For example, the tendency for longitudinal deformation under a longitudinally directed compressive load may increase with an increase in length, and decrease with an increase in width and wall thickness. Other variations in dimensions and/or shapes as may be anticipated by one of ordinary skill having the benefit of this disclosure are within the scope of this disclosure.

For example, in an instance where the kit 900 is dropped onto a hard floor surface in an orientation so that the proximal end of sealed enclosure 910 is downward, the proximal end 921 of opposing flexible walls 911 may impact the floor. The impact may cause medical plug delivery device 100 to longitudinally accelerate. The longitudinal acceleration of medical plug delivery device 100 may in turn result in the mass of elongate medical plug 200 creating a longitudinally directed force between elongate medical plug 200 and medical plug delivery device 100. If the force is great enough, it may overcome the frictional engagement force resulting in displacement of elongate medical plug 200 relative to medical plug delivery device 100. However, in such a scenario, proximal portion 920 of sealed enclosure 910 may deform and absorb the energy of impact, thereby reducing the acceleration of medical plug delivery device 100, which in turn reduces the longitudinally directed force between elongate medical plug 200 and medical plug delivery device 100. In such a scenario, the reduction of force may result in no displacement of elongate medical plug 200 relative to medical plug delivery device 100. Deformation of proximal and distal portions 920, 930 may also absorb longitudinally directed energy when kit 900 is exposed to vibration and thus prevent displacement of elongate medical plug 200 relative to medical plug delivery device 100.

In some embodiments, the sealed enclosure 910 may comprise a vacuum 913 within an interior of the sealed enclosure 910. For example, a vacuum may be applied after one or more medical plug delivery systems 905 is disposed therein and as sealed enclosure 910 is sealed. Vacuum 913 may define a pressure difference such that the opposing flexible walls 911 apply a force on the components within sealed enclosure 910, such as on the medical plug delivery device 100. The force may facilitate constraint of the medical plug delivery device 100 in a predetermined (and/or initially disposed location and/or orientation) within the sealed enclosure 910. The location may be in a central region 925 so as to establish a predetermined length of proximal and distal portions 920, 930. The level of vacuum 913 may be such that when kit 900 is exposed to the environmental conditions described above, longitudinal displacement of medical plug delivery system 905 within sealed enclosure 910 is less than a predetermined limit. In other words, the level of vacuum 913 may be sufficient to maintain the established length of proximal and distal portions 920, 930 during shipping and other conditions. The level of vacuum 913 may also be sufficient to prevent any displacement of medical plug delivery system 905 during handling, shipping, or other shocks to the sealed enclosure 910. In some embodiments, the vacuum 913 level may be between 20 and 700 mmHg, including between 20 and 200 mmHg.

Any methods disclosed herein include one or more steps or actions for performing the described method. The method steps and/or actions may be interchanged with one another. In other words, unless a specific order of steps or actions is required for proper operation of the embodiment, the order and/or use of specific steps and/or actions may be modified. Moreover, sub-routines or only a portion of a method described herein may be a separate method within the scope of this disclosure. Stated otherwise, some methods may include only a portion of the steps described in a more detailed method.

Reference throughout this specification to "an embodiment" or "the embodiment" means that a particular feature, structure, or characteristic described in connection with that embodiment is included in at least one embodiment. Thus, the quoted phrases, or variations thereof, as recited throughout this specification are not necessarily all referring to the same embodiment.

Similarly, it should be appreciated by one of skill in the art with the benefit of this disclosure that in the above description of embodiments, various features are sometimes grouped together in a single embodiment, figure, or description thereof for the purpose of streamlining the disclosure. This method of disclosure, however, is not to be interpreted as reflecting an intention that any claim requires more features than those expressly recited in that claim. Rather, as the following claims reflect, inventive aspects lie in a combination of fewer than all features of any single foregoing disclosed embodiment. Thus, the claims following this Detailed Description are hereby expressly incorporated into this Detailed Description, with each claim standing on its own as a separate embodiment. This disclosure includes all permutations of the independent claims with their dependent claims.

Recitation in the claims of the term "first" with respect to a feature or element does not necessarily imply the existence of a second or additional such feature or element. It will be apparent to those having skill in the art that changes may be made to the details of the above-described embodiments without departing from the underlying principles of the present disclosure.

## Claims

1. A medical plug delivery system (905), comprising:
a medical plug holding device (100) that includes a housing defining a lumen (102) that extends from a proximal end (112) to a distal end (122) of the medical plug holding device, wherein the housing comprises a distal connector (132) configured to connect to a distal medical device (700) in communication with a patient; **characterized by**
an elongate medical plug (200; 200'; 200"; 200"') that is substantially cylindrical in shape and is disposed within the lumen (102) of the medical plug holding device (100), wherein the elongate medical plug (200) defines a channel (210; 210'; 210"; 210'") that extends from a proximal end (202) to a distal end (204) of the elongate medical plug (200), wherein said channel provides a fluid bypass pathway.

2. The medical plug delivery system of claim 1, wherein the lumen (102) is structured to frictionally engage the elongate medical plug (200) within the cavity until:
(i) a distally directed flow of fluid passes through the lumen (102) with sufficient force to overcome the engagement between the elongate medical plug (200) and the cavity; or
(ii) a distally directed force from a stylet (500) is applied to the medical plug (200) to overcome the engagement between the elongate medical plug (200) and the cavity.

3. The medical plug delivery system of claim 1 or claim 2, wherein the force applied to the medical plug (200) displaces the elongate medical plug (200) from the medical plug holding device (100) to a void within the patient.

4. The medical plug delivery system of any one of claims 1-3, wherein the lumen (102) comprises an annual reduced diameter portion configured to engage the medical plug, and
wherein the diameter of the annual reduced diameter portion is smaller than the diameter of the elongated medical plug.

5. The medical plug delivery system of any one of claims 1-4, wherein the channel (210) is disposed on the outer surface of the plug, and
wherein the channel (210) has an arc shape.

6. The medical plug delivery system of any one of claims 1-5, wherein the channel (210) is disposed within the elongate medical plug, and
wherein the channel (210) has a circular cross-section.

7. The medical plug delivery system of any one of claims 1-6, wherein the elongated medical plug (200; 200'; 200"; 200"') includes multiple channels (210'; 210"; 210'") that extend from the proximal end (202) to the distal end (204) of the plug.

8. The medical plug delivery system of any one of claims 1-7, wherein the elongated medical plug (200) includes at least one channel (210'; 210"; 210‴) disposed on the outer surface of the elongated medical plug and at least one channel (210'; 210"; 210‴) disposed within the medical plug.

9. A kit (900) for a medical plug delivery system (905), comprising:
the medical plug delivery system (905) of claim 1; and
an enclosure (910) comprising flexible walls (911) disposed around the medical plug delivery system (905), the flexible walls (911) configured to inhibit displacement of the elongate medical plug relative to the medical plug delivery device.

10. The kit of claim 9, wherein the displacement of the elongate medical plug (200) relative to the medical plug delivery device (100) is inhibited by preventing longitudinal acceleration of the medical plug delivery device.

11. The kit of any one of claims 9-10, wherein the enclosure (910) comprises a proximal portion (920) and a distal portion (930) and wherein the medical plug delivery system (905) is disposed centrally between the proximal and distal portions.

12. The kit of claim 11, wherein a longitudinal axis of the medical plug delivery system is aligned with a longitudinal axis of the enclosure (910).

13. The kit of claim 11, wherein the proximal (920) and distal portions (930) are configured to deform under a compressive load.

14. The kit of any one of claims 9-13, wherein the enclosure (910) is configured to constrain relative displacement between the medical plug delivery system (905) and the flexible walls (911).

15. The kit of any one of claims 9-14, wherein the flexible wall (911) comprise opposing flexible walls (911) and the kit comprises a pressure difference across each opposing flexible wall such that the pressure difference is configured to frictionally couple the medical plug delivery system to the opposing flexible walls.

## Patentansprüche

1. System (905) zum Einführen eines medizinischen Stopfens, umfassend:
eine Vorrichtung (100) zum Halten eines medizinischen Stopfens, die ein Gehäuse umfasst, das ein Lumen (102) definiert, das sich von einem proximalen Ende (112) zu einem distalen Ende (122) der Vorrichtung zum Halten eines medizinischen Stopfens erstreckt, wobei das Gehäuse einen distalen Verbinder (132) umfasst, der eingerichtet ist, sich mit einer distalen medizinischen Vorrichtung (700), die Verbindung mit einem Patienten steht, zu verbinden; **gekennzeichnet durch**
einen länglichen medizinischen Stopfen (200; 200'; 200"; 200‴), der eine im Wesentlichen zylindrische Form aufweist und innerhalb des Lumens (102) der Vorrichtung (100) zum Halten eines medizinischen Stopfens angeordnet ist, wobei der längliche medizinische Stopfen (200) einen Kanal (210; 210'; 210"; 210‴) definiert, der sich von einem proximalen Ende (202) zu einem distalen Ende (204) des länglichen medizinischen Stopfens (200) erstreckt, wobei der Kanal einen Fluidumgehungspfad bereitstellt.

2. System (905) zum Einführen eines medizinischen Stopfens nach Anspruch 1, wobei das Lumen (102) so strukturiert ist, dass es mit dem länglichen medizinischen Stopfen (200) in der Hohlraum in Reibungseingriff kommt, bis
(i) ein distal gerichteter Flüssigkeitsstrom mit ausreichender Kraft durch das Lumen (102) fließt, um den Eingriff zwischen dem länglichen medizinischen Stopfen (200) und dem Hohlraum zu überwinden; oder
(ii) eine distal gerichtete Kraft von einem Stilett (500) auf den medizinischen Stopfen (200) ausgeübt wird, um den Eingriff zwischen dem länglichen medizinischen Stopfen (200) und dem Hohlraum zu überwinden.

3. System (905) zum Einführen eines medizinischen Stopfens nach Anspruch 1 oder Anspruch 2, wobei die auf den medizinischen Stopfen (200) ausgeübte Kraft den länglichen medizinischen Stopfen (200) von der Vorrichtung (100) zum Halten eines medizinischen Stopfens zu einem Hohlraum im Patienten verschiebt.

4. System (905) zum Einführen eines medizinischen Stopfens nach einem der Ansprüche 1-3, wobei das Lumen (102) einen Abschnitt mit reduziertem Durchmesser umfasst, der eingerichtet ist, mit dem medizinischen Stopfen in Eingriff zu kommen, und wobei der Durchmesser des Abschnitts mit reduziertem Durchmesser kleiner ist als der Durchmesser des länglichen medizinischen Stopfens.

5. System (905) zum Einführen eines medizinischen Stopfens nach einem der Ansprüche 1-4, wobei der Kanal (210) auf der Außenfläche des Stopfens angeordnet ist und
wobei der Kanal (210) eine Bogenform aufweist.

6. System (905) zum Einführen eines medizinischen Stopfens nach einem der Ansprüche 1-5, wobei der Kanal (210) innerhalb des länglichen medizinischen Stopfens angeordnet ist und
wobei der Kanal (210) einen kreisförmigen Querschnitt aufweist.

7. System (905) zum Einführen eines medizinischen Stopfens nach einem der Ansprüche 1-6, wobei der längliche medizinische Stopfen (200; 200'; 200"; 200‴) mehrere Kanäle (210'; 210"; 210‴) umfasst, die sich vom proximalen Ende (202) zum distalen Ende (204) des Stopfens erstrecken.

8. System (905) zum Einführen eines medizinischen Stopfens nach einem der Ansprüche 1-7, wobei der längliche medizinische Stopfen (200) mindestens einen Kanal (210'; 210"; 210‴) aufweist, der auf der Außenfläche des länglichen medizinischen Stopfens angeordnet ist, und mindestens einen Kanal (210'; 210"; 210‴) aufweist, der innerhalb des medizinischen Stopfens angeordnet ist.

9. Kit (900) für ein System (905) zum Einführen eines medizinischen Stopfens, umfassend:
das System (905) zum Einführen eines medizinischen Stopfens nach Anspruch 1; und eine Einfassung (910), umfassend flexible Wände (911), die um das System (905) zum Einführen eines medizinischen Stopfens herum angeordnet sind, wobei die flexiblen Wände (911) eingerichtet sind, eine Verschiebung des länglichen medizinischen Stopfens relativ zur Vorrichtung zum Einführen eines medizinischen Stopfens zu verhindern.

10. Kit nach Anspruch 9, wobei die Verschiebung des länglichen medizinischen Stopfens (200) relativ zur Vorrichtung (100) zum Einführen eines medizinischen Stopfens durch Verhindern einer Längsbeschleunigung der Vorrichtung zum Einführen eines medizinischen Stopfens gehemmt wird.

11. Kit nach einem der Ansprüche 9-10, wobei die Einfassung (910) einen proximalen Abschnitt (920) und einen distalen Abschnitt (930) umfasst und wobei das System (905) zum Einführen eines medizinischen Stopfens zentral zwischen dem proximalen und dem distalen Abschnitt angeordnet ist.

12. Kit nach Anspruch 11, wobei eine Längsachse des Systems zum Einführen eines medizinischen Stopfens mit einer Längsachse der Einfassung (910) ausgerichtet ist.

13. Kit nach Anspruch 11, wobei der proximale (920) und der distale Abschnitt (930) eingerichtet sind, sich unter einer Druckbelastung zu verformen.

14. Kit nach einem der Ansprüche 9-13, wobei die Einfassung (910) eingerichtet ist, die relative Verschiebung zwischen dem System (905) zum Einführen eines medizinischen Stopfens und den flexiblen Wänden (911) einzuschränken.

15. Kit nach einem der Ansprüche 9-14, wobei die flexible Wände (911) gegenüberliegende flexible Wände (911) umfassen und der Kit eine Druckdifferenz über jede gegenüberliegende flexible Wand umfasst, so dass die Druckdifferenz eingerichtet ist, das System zum Einführen eines medizinischen Stopfens reibschlüssig mit den gegenüberliegenden flexiblen Wänden zu verbinden.

## Revendications

1. Système de pose de tampon médical (905), comprenant :
un dispositif porteur de tampon médical (100) qui comprend un logement définissant une lumière (102) qui s'étend d'une extrémité proximale (112) à une extrémité distale (122) du dispositif porteur de tampon médical, dans lequel le logement comprend un élément de liaison distal (132) configuré pour s'associer à un dispositif médical distal (700) en communication avec un patient ; **caractérisé par**
un tampon médical allongé (200 ; 200'; 200" ; 200'") qui est de forme sensiblement cylindrique et qui est disposé à l'intérieur de la lumière (102) du dispositif porteur de tampon médical (100), dans lequel le tampon médical allongé (200) définit un canal (210 ; 210' ; 210" ; 210'") qui s'étend d'une extrémité proximale (202) à une extrémité distale (204) du tampon médical allongé (200), dans lequel ledit canal fournit un trajet de dérivation de fluide.

2. Système de pose de tampon médical selon la revendication 1, dans lequel la lumière (102) est structurée pour être en prise par frottement avec le tampon médical allongé (200) à l'intérieur de la cavité jusqu'à ce que :
(i) un écoulement de fluide dirigé de manière distale passe à travers la lumière (102) avec une force suffisante pour contrer la mise en prise entre le tampon médical allongé (200) et la cavité ; ou
(ii) une force dirigée de manière distale à partir d'un stylet (500) est appliquée au tampon médical (200) pour contrer la mise en prise entre le tampon médical allongé (200) et la cavité.

3. Système de pose de tampon médical selon la revendication 1 ou la revendication 2, dans lequel la force appliquée au tampon médical (200) déplace le tampon médical allongé (200) du dispositif porteur de tampon médical (100) jusqu'à un vide à l'intérieur du patient.

4. Système de pose de tampon médical selon l'une quelconque des revendications 1 à 3, dans lequel la lumière (102) comprend une partie annulaire de diamètre réduit configurée pour être en prise avec le tampon médical, et
dans lequel le diamètre de la partie annulaire de diamètre réduit est inférieur au diamètre du tampon médical allongé.

5. Système de pose de tampon médical selon l'une quelconque des revendications 1 à 4, dans lequel le canal (210) est disposé sur la surface externe du tampon, et
dans lequel le canal (210) a une forme d'arc.

6. Système de pose de tampon médical selon l'une quelconque des revendications 1 à 5, dans lequel le canal (210) est disposé à l'intérieur du tampon médical allongé, et
dans lequel le canal (210) a une section transversale circulaire.

7. Système de pose de tampon médical selon l'une quelconque des revendications 1 à 6, dans lequel le tampon médical allongé (200 ; 200' ; 200" ; 200"') comprend une pluralité de canaux (210'; 210" ; 210'") qui s'étendent de l'extrémité proximale (202) à l'extrémité distale (204) du tampon.

8. Système de pose de tampon médical selon l'une quelconque des revendications 1 à 7, dans lequel le tampon médical allongé (200) comprend au moins un canal (210' ; 210" ; 210'") disposé sur la surface externe du tampon médical allongé et au moins un canal (210' ; 210" ; 210'") disposé à l'intérieur du tampon médical.

9. Kit (900) pour un système de pose de tampon médical (905), comprenant :
le système de pose de tampon médical (905) selon la revendication 1 ; et
une enceinte (910) comprenant des parois flexibles (911) disposées autour du système de pose de tampon médical (905), les parois flexibles (911) étant configurées pour empêcher le déplacement du tampon médical allongé par rapport au dispositif de pose de tampon médical.

10. Kit selon la revendication 9, dans lequel le déplacement du tampon médical allongé (200) par rapport au dispositif de pose de tampon médical (100) est bloqué par empêchement de l'accélération longitudinale du dispositif de pose de tampon médical.

11. Kit selon l'une quelconque des revendications 9 à 10, dans lequel l'enceinte (910) comprend une partie proximale (920) et une partie distale (930) et dans lequel le système de pose de tampon médical (905) est disposé de manière centrale entre les parties proximale et distale.

12. Kit selon la revendication 11, dans lequel un axe longitudinal du système de pose de tampon médical est aligné avec un axe longitudinal de l'enceinte (910).

13. Kit selon la revendication 11, dans lequel les parties proximale (920) et distale (930) sont configurées pour se déformer sous une charge de compression.

14. Kit selon l'une quelconque des revendications 9 à 13, dans lequel l'enceinte (910) est configurée pour contraindre un déplacement relatif entre le système de pose de tampon médical (905) et les parois flexibles (911).

15. Kit selon l'une quelconque des revendications 9 à 14, dans lequel les parois flexibles (911) comprennent des parois flexibles opposées (911) et le kit comprend une différence de pression à travers chaque paroi flexible opposée de sorte que la différence de pression est configurée pour coupler par frottement le système de pose de tampon médical aux parois flexibles opposées.
